# FASCICULE DE BREVET EUROPEEN

(11) **EP 3 946 237 B1**
(45) Date de publication et mention de la délivrance du brevet: **16.07.2025**
(21) Numéro de dépôt: 20713027.9
(22) Date de dépôt: 27.03.2020
(51) Int. Cl.: A61K 8/49, A61Q 19/00, A61Q 5/00, A61Q 15/00, A01N 43/00, A01N 43/90

(54) **UTILISATION DE DIANHYDROHEXITOL POUR LA CONSERVATION DES PRÉPARATIONS COSMÉTIQUES**
VERWENDUNG VON DIANHYDROHEXITOL ZUR KONSERVIERUNG VON KOSMETISCHEN ZUBEREITUNGEN
USE OF DIANHYDROHEXITOL FOR PRESERVING COSMETIC PREPARATIONS

(30) Priorité: 28.03.2019 FR 1903292
(43) Date de publication de la demande: 09.02.2022
(73) Titulaire: ROQUETTE FRERES, 62136 Lestrem (FR)
(72) Inventeur: MENTINK, Léon, 59800 Lille (FR); WILS, Daniel, 59190 Morbecque (FR)
(74) Mandataire: Plasseraud IP
(86) Numéro de dépôt international: PCT/EP2020/058680
(87) Numéro de publication internationale: WO 2020/193744

(56) Documents cités:
- WO-A1-2016/156505
- WO-A1-2019/025730
- WO-A1-2019/106316
- US-A1- 2016 354 296

## Description

### Domaine technique

La présente demande se situe dans le domaine de la cosmétique, plus précisément dans le domaine des agents de conservation antimicrobiens et/ou bactériostatiques et/ou bactéricides, et/ou antifongiques, préférentiellement sur les souches bactériennes et/ou fongiques présentes dans les préparations cosmétiques ou dermatologiques. Préférentiellement, il est proposé une utilisation d'isosorbide pour réduire ou éliminer les effets de la dégradation de ladite préparation suite à une contamination par des microorganismes du microbiome cutané humain ou présents dans l'atmosphère. L'isosorbide est utilisé seul ou en complément d'autres agents conservateurs déjà employés à cet effet.

### Technique antérieure

Dans le brevet US9295626 de Pilz et al., une méthode de conservation des préparations cosmétiques, dermatologiques et pharmaceutiques par l'utilisation de composés bactéricides à base de monoester et/ou diester d'isosorbide est dévoilée. Ce brevet présente une concentration minimale inhibitrice pour l'isosorbide égale à 10 % sur de nombreuses souches (col.27, tableau 2), dont notamment les souches suivantes que l'on peut retrouver dans le microbiome cutané : *Aspergillus brasiliensis, Candida albicans, Staphylococcus aureus.*

Le brevet US9993000 de Stoer et al. divulgue également l'utilisation de dérivés d'isosorbide, ici des éthers, en tant qu'agent bactéricide pour préserver les compositions cosmétiques, notamment en inhibant la croissance d'une multitude de souche bactériennes, dont Propionobacterium acnes.

### Problème technique

La peau humaine est colonisée par une flore bactérienne résidente, qui constitue le microbiome cutané. Bien que cette flore soit la plupart du temps non pathogène, il peut y avoir des situations anormales où elle le devient. Une première situation anormale est celle d'une contamination du microbiome cutané par un microorganisme extérieur pathogène, par contact avec un environnement contaminé. Une seconde situation anormale est celle de l'apparition d'un déséquilibre dans les interactions entre les microorganismes constitutifs du microbiome cutané, qui résulte en la prolifération d'un microorganisme au détriment des autres microorganismes. Une telle prolifération peut être généralisée sur une grande surface de la peau, ou peut-être plus localisée, par exemple dans les zones de la peau plus riches en eau et plus chaudes, ou par exemple sur le visage ou scalp en raison de la présence de réserves nutritives pour lesdits microorganismes, tels que les triglycérides localisés dans les glandes à la base des cheveux ou des poils. En-dehors des conséquences potentiellement néfastes pour la santé de la peau ou de l'individu, ces situations anormales provoquent aussi des désagréments d'ordre cosmétique, notamment visuels, tactiles, ou olfactifs, qui peuvent nuire au confort de l'individu ou à son image, et ainsi perturber ou dégrader sa vie sociale.

Une préparation cosmétique ou dermatologique est généralement conditionnée de manière aseptique dans un contenant hermétique disposant d'un orifice par lequel ledit produit est expulsé, usuellement par action mécanique, afin d'être appliqué sur le corps humain. Cette opération d'expulsion conduit systématiquement à la mise en contact du produit cosmétique ou dermatologique avec l'atmosphère ou le corps humain, et notamment avec le microbiome topique humain, principalement le microbiome cutané. Cette mise en contact peut induire une contamination de ladite préparation cosmétique ou dermatologique par des microorganismes de nature très variée : bactéries, levures, champignons. Ces microorganismes peuvent trouver dans la préparation cosmétique ou dermatologique des substances organiques nutritives et de l'eau, et ainsi s'y développer de manière incontrôlée. Une telle prolifération de microorganismes est à éviter car ces microorganismes sont potentiellement pathogènes, et peuvent dans une moindre mesure par leur développement, modifier la préparation cosmétique ou dermatologique de telle sorte qu'elle devienne inactive ou que son utilisation devienne déplaisante, notamment du fait de l'apparition de mauvaises odeurs ou d'un changement de texture ou du profil sensoriel.

Il y a de nos jours un besoin accru des consommateurs d'avoir des produits d'origine naturelle. L'utilisation selon l'invention répond à ce besoin, en proposant d'utiliser au moins dianhydrohexitol, car les dianhydrohexitols sont bien des molécules d'origine naturelle, produits à partir d'amidon d'origine céréalière par exemple.

### Description des modes de réalisation

Il est du mérite de la demanderesse d'avoir découvert, de manière inattendue, que les dianhydrohexitols non dérivés, préférentiellement l'isosorbide, ont des effets antimicrobiens et/ou bactéricides et/ou bactériostatiques, et/ou antifongiques, sur des souches microbiennes responsables de la contamination et la dégradation physicochimique des préparations cosmétiques ou dermatologiques.

L'invention a plus particulièrement attrait à l'utilisation d'au moins un dianhydrohexitol dans une préparation cosmétique ou dermatologique à usage topique pour limiter ou empêcher la prolifération de micro-organismes du microbiome cutané humain.

Il est aussi proposé une utilisation d'isosorbide comme agent de conservation des préparations cosmétiques ou dermatologiques, seul ou en complément d'un autre agent de conservation connu

### Utilisation de dianhydrohexitol en conservation de préparations cosmétiques

La présente invention concerne l'utilisation d'au moins un dianhydrohexitol dans une préparation cosmétique ou dermatologique à usage topique pour limiter ou empêcher la prolifération de micro-organismes du microbiome cutané humain ou présents dans l'atmosphère.

Préférentiellement, les micro-organismes sont choisis parmi les bactéries et les champignons, et sont de préférence à l'origine de l'inactivation d'ingrédients actifs cosmétiques, et/ou de l'apparition de mauvaises odeurs, et/ou de changement de texture d'une préparation cosmétique ou dermatologique.

Selon un mode de réalisation préférentiel, le dianhydrohexitol est choisi parmi l'isosorbide, l'isomannide, l'isoidide, préférentiellement l'isosorbide.

De manière additionnelle, l'utilisation dans une préparation cosmétique ou dermatologique est faite à une teneur en dianhydrohexitol de 0,1 % à 50 % en poids, préférentiellement de 0,5 % à 25 % en poids, plus préférentiellement de 1 % à 25 % en poids, encore plus préférentiellement de 2 % à 15 % en poids, et tout préférentiellement de 5 % à 9 % en poids, par rapport au poids total de la composition.

Selon un mode de réalisation de l'utilisation selon l'invention, le dianhydrohexitol est un agent antimicrobien et/ou bactéricide et/ou bactériostatique et/ou antifongique, et permet de réduire ou empêcher le développement de souches bactériennes et/ou fongiques issues du microbiome topique humain, dans ladite préparation cosmétique ou dermatologique.

Préférentiellement, la souche bactérienne est choisie dans la famille *Propionobacterium spp,* préférentiellement la souche bactérienne est *Propionobacterium acnes* ; et/ou la souche bactérienne est choisie dans la famille *Corynebacterium spp,* préférentiellement la souche bactérienne *Corynebacterium xerosis* ; et/ou la souche bactérienne est choisie dans la famille *Staphylococcus spp*, préférentiellement la souche bactérienne est *Staphylococcus epidermis* ; et la souche fongique est choisie dans la famille *Malassezia spp,* préférentiellement la souche fongique est *Malassezia furfur.*

Selon une variante de l'utilisation selon l'invention, le dianhydrohexitol est utilisé en combinaison à d'autres biocides couramment employés dans les préparations cosmétiques ou dermatologiques, tels que les acides, les sels alcalins d'acide benzoïque ou d'acide sorbique ou d'acide propionique ; ou d'autre biocides tels que le phénoxyéthanol ou la chlorphenesine.

### Dianhydrohexitol

La solution aqueuse en question peut contenir un dianhydrohexitol seul, comme elle peut en contenir plusieurs. Ces dianhydrohexitols (1,4 - 3,6-dianhydrohexitols) sont l'isosorbide (1,4-3,6-dianhydrosorbitol), l'isomannide (1,4-3,6-dianhydromannitol), l'isoidide (1,4-3,6-dianhydroiditol) et les mélanges d'au moins deux de ces produits. De préférence, la solution aqueuse ne contient qu'un seul dianhydrohexitol qui est l'isosorbide.

A cet égard, la Demanderesse indique que généralement, les dianhydrohexitols sont synthétisés en présence d'eau (ou de l'eau est générée au cours de leur synthèse) : en récupérant ledit dianhydrohexitol dans ce milieu réactionnel, on dispose immédiatement d'une composition sous forme de solution aqueuse de dianhydrohexitol utilisable selon l'invention. Les solutions de dianhydrohexitols peuvent notamment être obtenues selon les procédés décrits dans les demandes de brevet précitées EP 1 287 000 et WO 03 / 043959. On peut choisir de conserver tout ou partie de l'eau utilisée lors de la préparation du dianhydrohexitol ou d'éliminer en totalité l'eau pour obtenir un produit sous forme solide qu'on remettra en solution aqueuse par simple ajout d'eau, ce qui constitue une autre possibilité pour préparer une solution aqueuse de dianhydrohexitol utilisable selon l'invention.

La demanderesse précise que le terme « 1,4-3,6-dianhydrohexitol » n'inclut pas les dérivés de 1,4-3,6-dianhydrohexitol, notamment tels que les éthers ou esters de 1,4-3,6-dianhydrohexitol.

### Préparation cosmétique ou dermatologique

Par « préparation cosmétique ou dermatologique », la demanderesse entend toute composition destinée à être mise en contact avec la peau, humaine ou animale.

La préparation cosmétique ou dermatologique selon l'invention comprend comme agent actif de conservation, notamment antibactérien et/ou antifongique, au moins un dianhydrohexitol, préférentiellement choisi parmi l'isosorbide, l'isomannide, l'isoidide, préférentiellement est l'isosorbide.

Selon un mode de réalisation préférentiel, la préparation cosmétique ou dermatologique selon l'invention comprend de 0,1 % à 50 % en poids de dianhydrohexitol, préférentiellement de 0,5 à 25 %, plus préférentiellement de 1 % à 25 %, encore plus préférentiellement de 2 % à 15 %, et tout préférentiellement de 5 % à 9 %.

Selon un mode de réalisation très préférentiel, la préparation cosmétique ou dermatologique contient comme seul agent antimicrobien et/ou bactéricide et/ou bactériostatique et/ou antifongique, au moins un dianhydrohexitol, préférentiellement choisi parmi l'isosorbide, l'isomannide, l'isoidide, préférentiellement est l'isosorbide.

La préparation cosmétique ou dermatologique selon l'invention permet de réduire ou éliminer l'inactivation des actifs cosmétiques du fait d'une dégradation microbienne, et/ou de réduire ou éliminer l'apparition de mauvaises odeurs, ou le changement de texture de ladite préparation cosmétique ou dermatologique.

La préparation cosmétique peut être un produit pour la peau, un produit pour les cheveux, un maquillage, ou un produit d'hygiène.

Parmi les produits de soin pour la peau, la préparation cosmétique selon l'invention peut être choisie parmi les crèmes de jour, les crèmes solaires, les crèmes après-soleil, les autobronzants, les masques. Parmi les produits de soin capillaires, la préparation cosmétique selon l'invention est préférentiellement choisie parmi les shampoings, les après-shampoings (crèmes, masques, lotions), les produits de coiffage (spray, gels, cires), les produits de coloration. Parmi les produits de maquillage, la préparation cosmétique selon l'invention est préférentiellement choisie parmi les fonds de teints, les fards à paupières. Parmi les produits d'hygiène, la préparation cosmétique selon l'invention est préférentiellement choisie parmi les gels lavant, les gels douches, les lingettes nettoyantes ou démaquillantes, les solutions ou gels hydroalcooliques, les savons, les déodorants, les anti-transpirants, les sprays pour le corps.

### Exemple

### Mesures in-vitro de l'effet de l'isosorbide sur des souches microbiennes

Les échantillons sont préparés en conditions stériles et déposés dans des microplaques (puits de 2ml) selon les concentrations ci-dessous. Deux témoins d'analyse ont été réalisés :
- un témoin positif correspondant à du Phenonip^{®} 0,5%;
- un témoin négatif constitué de Sérum physiologique 0,85%.

Échantillons et témoins sont contaminés par trois souches bactériennes, à savoir *Staphylococcus epidermidis, Corynebacterium xerosis,* et *Propionobacterium acnes,* à raison d'environ 1,43.105 à 4,15.105 unités formant colonies par millilitre, noté ufc/ml ; et une souche fongique, à savoir *Malassezia furfur,* à raison d'environ 1,50.104 à 3,80.104 ufc/ml. A l'issue de la contamination, les échantillons sont soigneusement mélangés par cycles d'aspirations - refoulements afin d'assurer une répartition homogène du micro-organisme. L'ensemble est incubé à 22°C pendant 28 jours.

Des prélèvements et dénombrements de la population microbienne sont effectués à 24 heures, 7, 14, 21 et 28 jours pour chaque souche microbienne. Les échantillons contaminés sont prélevés puis déposés en dilutions sériées dans des plaques de microtitration, en présence de milieu de culture, qui est une solution saline à 0,85% en chlorure de sodium, et d'un réactif révélateur de l'activité déshydrogénase, qui est le chlorure de 2,3,5-triphenyltetrazolium (noté TTC). Les résultats sont présentés dans les pages suivantes avec l'évolution de la population microbienne au cours des 28 jours d'étude, pour la souche microbienne testée.

Les mesures de populations microbiennes dans les prélèvements faits à chaque temps, sont réalisées selon la méthode de microtitration suivante, pour un prélèvement. Dans les 96 puits de volume 250 µL d'une plaque de microtitration, 20 µL du prélèvement sont dilués d'un facteur 10 par dispersion dans 180 µL de bouillon Letheen (Difco, ref 268110) contenant 1,5% de Tween 80 (Sigma, ref P1754) and le TTC (Sigma, ref T8877). La microplaque est incubée 48 heures à 32,5°C, et la croissance des microorganismes est suivie par le changement de couleur, d'incolore à rouge/rose. La dilution réciproque la plus élevée indiquant une croissance permet la détermination du nombre de log de chaque microorganisme à chaque temps.

Les mesures de populations microbiennes réalisées à chaque temps de prélèvement, sont exprimées en unités formant colonies/ml.

**[Tableau 1]**

| Effet bactéricide de l'isosorbide sur la souche *Propionobacterium acnes* | | | | | | |
|---|---|---|---|---|---|---|
| | | Nombre de souche formant colonies après : | | | | |
| Dose | Innoculum | 24 h | 7 jours | 14 jours | 21 jours | 28 jours |
| Témoin positif | 3,98.10⁵ | 4,00.10³ | 7,00.10⁴ | 4,00.10⁴ | 1,00.10⁴ | 1,00.10⁴ |
| Témoin négatif | 3,98.10⁵ | 0 | 0 | 0 | 0 | 0 |
| 1 % | 3,98.10⁵ | 0 | 0 | 0 | 0 | 0 |
| 5% | 3,98.10⁵ | 0 | 0 | 0 | 0 | 0 |
| 9% | 3,98.10⁵ | 0 | 0 | 0 | 0 | 0 |

Après 24 heures, aucune souche formant colonie n'est observée : l'isosorbide a permis de tuer la totalité de l'inoculum. L'isosorbide est un bactéricide de la souche *Propionobacterium acnes.*

**[Tableau 2]**

| Effet antifongique de l'isosorbide sur la souche *Malassezia furfur* | | | | | | |
|---|---|---|---|---|---|---|
| | | Nombre de souche formant colonies après : | | | | |
| Dose d'isosorbide | Innoculum | 24 h | 7 jours | 14 jours | 21 jours | 28 jours |
| Témoin positif | 1,5.10⁴ | 4,00.10³ | 1,00.10³ | 7,00.10² | 1,00.10³ | 1,00.10³ |
| Témoin négatif | 1,5.10⁴ | 0 | 0 | 0 | 0 | 0 |
| 1 % | 1,5.10⁴ | 0 | 0 | 0 | 0 | 0 |
| 5% | 1,5.10⁴ | 0 | 0 | 0 | 0 | 0 |
| 9% | 1,5.10⁴ | 0 | 0 | 0 | 0 | 0 |

Après 24 heures, aucune souche formant colonie n'est observée: l'isosorbide a permis de tuer la totalité de l'inoculum. L'isosorbide est un bactéricide de la souche *Malassezia furfur.*

**[Tableau 3]**

| Effet bactéricide de l'isosorbide sur la souche *Corynobacterium xerosis* | | | | | | |
|---|---|---|---|---|---|---|
| | | Nombre de souche formant colonies après : | | | | |
| Dose | Innoculum | 24 h | 7 jours | 14 jours | 21 jours | 28 jours |
| Témoin positif | 3,2.10⁵ | 7,00.10⁴ | 1,00.10⁴ | 7,00.10³ | 4,00.10³ | 7,00.10³ |
| Témoin négatif | 3,2.10⁵ | 0 | 0 | 0 | 0 | 0 |
| 1 % | 3,2.10⁵ | 4.10² | 0 | 0 | 0 | 0 |
| 5% | 3,2.10⁵ | 1.10² | 0 | 0 | 0 | 0 |
| 9% | 3,2.10⁵ | 3,7.10² | 0 | 0 | 0 | 0 |

Après 24 heures, le nombre de souche formant colonie est réduit d'au moins trois log de 10 pour les trois doses testées. Après 7 jours, il n'y a plus aucune souche formant colonie : l'isosorbide est un bactéricide modéré de la souche *Corynobacterium xerosis.*

**[Tableau 4]**

| Effet bactéricide de l'isosorbide sur la souche *Staphylococcus epidermis* | | | | | | |
|---|---|---|---|---|---|---|
| | | Nombre de souche formant colonies après : | | | | |
| Dose | Innoculum | 24 h | 7 jours | 14 jours | 21 jours | 28 jours |
| Témoin positif | 1,43.10⁵ | 7,00.10⁴ | 4,00.10⁴ | 7,00.10⁴ | 1,00.10⁵ | 1,00.10⁵ |
| Témoin négatif | 1,43.10⁵ | 0 | 0 | 0 | 0 | 0 |
| 1 % | 1,43.10⁵ | 4.10³ | 0 | 0 | 0 | 0 |
| 5% | 1,43.10⁵ | 1.10³ | 0 | 0 | 0 | 0 |
| 9% | 1,43.10⁵ | 7.10² | 0 | 0 | 0 | 0 |

Après 24 heures, le nombre de souche formant colonie est réduit d'au moins deux log de 10 pour les trois doses testées. Après 7 jours, il n'y a plus aucune souche formant colonie : l'isosorbide est un bactéricide faible de la souche *Staphylococcus epidermis.*

L'invention ne se limite pas aux exemples décrits ci-avant, seulement à titre d'exemple, mais elle englobe toutes les variantes que pourra envisager l'homme de l'art dans le cadre de la protection recherchée.

## Revendications

1. Utilisation d'au moins un dianhydrohexitol pour limiter ou empêcher la prolifération de micro-organismes du microbiome cutané humain dans une préparation cosmétique ou dermatologique à usage topique.

2. Utilisation selon la revendication précédente, **caractérisée en ce que** les micro-organismes sont choisis parmi les bactéries et les champignons, et sont de préférence à l'origine de l'inactivation d'ingrédients actifs cosmétiques, et/ou de l'apparition de mauvaises odeurs, et/ou de changement de texture d'une préparation cosmétique ou dermatologique.

3. Utilisation selon l'une des revendications précédentes, **caractérisée en ce que** le dianhydrohexitol est choisi parmi l'isosorbide, l'isomannide, l'isoidide, préférentiellement l'isosorbide.

4. Utilisation selon l'une des revendications précédentes, dans une préparation cosmétique ou dermatologique en une teneur en dianhydrohexitol de 0,1 % à 50 % en poids, préférentiellement de 0,5 % à 25 % en poids, plus préférentiellement de 1 % à 25 % en poids, encore plus préférentiellement de 2 % à 15 % en poids, et tout préférentiellement de 5 % à 9 % en poids, par rapport au poids total de la composition.

5. Utilisation selon l'une des revendications précédentes, **caractérisée en ce que** ledit au moins un dianhydrohexitol est un agent bactériostatique ou/et bactéricide et/ou antifongique, et qu'il permet de réduire ou empêcher le développement de souches bactériennes et/ou fongiques issues du microbiome topique humain, dans ladite préparation cosmétique ou dermatologique.

6. Utilisation selon l'une des revendications 2 à 5, **caractérisée en ce que** la souche bactérienne est choisie dans la famille *Propionobacterium spp,* préférentiellement la souche bactérienne est *Propionobacterium* acnes ; et/ou la souche bactérienne est choisie dans la famille *Corynebacterium spp,* préférentiellement la souche bactérienne *Corynebacterium xerosis ;* et/ou la souche bactérienne est choisie dans la famille *Staphylococcus spp,* préférentiellement la souche bactérienne est *Staphylococcus epidermis* ; et **en ce que** la souche fongique est choisie dans la famille *Malassezia spp,* préférentiellement la souche fongique est *Malassezia furfur.*

## Patentansprüche

1. Verwendung wenigstens eines Dianhydrohexitols zur Einschränkung oder Verhinderung der Vermehrung von Mikroorganismen des menschlichen Hautmikrobioms in einer kosmetischen oder dermatologischen Zubereitung zur topischen Anwendung.

2. Verwendung nach dem vorhergehenden Anspruch, **dadurch gekennzeichnet, dass** die Mikroorganismen aus Bakterien und Pilzen gewählt sind und bevorzugt die Inaktivierung kosmetischer Wirkstoffe und/oder das Auftreten von schlechten Gerüchen und/oder eine Veränderung der Textur einer kosmetischen oder dermatologischen Zubereitung bewirken.

3. Verwendung nach einem der vorhergehenden Ansprüche, **dadurch gekennzeichnet, dass** das Dianhydrohexitol aus Isosorbid, Isomannid, Isoidid, bevorzugt Isosorbid gewählt ist.

4. Verwendung nach einem der vorhergehenden Ansprüche in einer kosmetischen oder dermatologischen Zubereitung mit einem Gehalt an Dianhydrohexitol von 0,1 bis 50 Gew.-%, bevorzugt 0,5 bis 25 Gew.-%, stärker bevorzugt 1 bis 25 Gew.-%, noch stärker bevorzugt 2 bis 15 Gew.-% und am stärksten bevorzugt 5 bis 9 Gew.-%, bezogen auf das Gesamtgewicht der Zusammensetzung.

5. Verwendung nach einem der vorhergehenden Ansprüche, **dadurch gekennzeichnet, dass** das wenigstens eine Dianhydrohexitol ein bakteriostatisches oder/und bakterizides und/oder fungizides Mittel ist und die Entwicklung von Bakterienstämmen und/oder Pilzen aus dem menschlichen topischen Mikrobiom in der kosmetischen oder dermatologischen Zubereitung reduziert oder verhindert.

6. Verwendung nach einem der Ansprüche 2 bis 5, **dadurch gekennzeichnet, dass** der Bakterienstamm aus der Familie *Propionobacterium spp* gewählt ist, wobei der Bakterienstamm bevorzugt *Propionobacterium acnes* ist; und/oder der Bakterienstamm aus der Familie *Corynebacterium spp* gewählt ist, wobei der Bakterienstamm bevorzugt *Corynebacterium xerosis* ist; und/oder der Bakterienstamm aus der Familie *Staphylococcus spp* gewählt ist, wobei der Bakterienstamm bevorzugt *Staphylococcus epidermis* ist; und dadurch, dass der Pilzstamm aus der Familie *Malassezia spp* gewählt ist, wobei der Pilzstamm bevorzugt *Malassezia furfur* ist.

## Claims

1. A use of at least one dianhydrohexitol to limit or prevent the proliferation of microorganisms of the human cutaneous microbiome in a cosmetic or dermatological preparation for topical use.

2. The use according to the preceding claim, **characterised in that** the microorganisms are selected from among bacteria and fungi, and are preferably at the origin of the inactivation of cosmetic active ingredients, and/or the apparition of bad odours, and/or a change in texture of a cosmetic or dermatological preparation.

3. The use according to one of the preceding claims, **characterised in that** the dianhydrohexitol is selected from among isosorbide, isomannide, isoidide, preferably isosorbide.

4. The use according to one of the preceding claims, in a cosmetic or dermatological preparation with a dianhydrohexitol content of 0.1% to 50% by weight, preferably 0.5% to 25% by weight, more preferably 1% to 25% by weight, even more preferably 2% to 15% by weight, and most preferably 5% to 9% by weight, relative to the total weight of the composition.

5. The use according to one of the preceding claims, **characterised in that** said at least one dianhydrohexitol is a bacteriostatic and/or bactericidal and/or antifungal agent, and that it allows reducing or preventing the development of bacterial and/or fungal strains derived from the human topical microbiome, in said cosmetic or dermatological preparation.

6. The use according to one of claims 2 to 5, **characterised in that** the bacterial strain is selected from among the *Propionobacterium spp* family, preferably the bacterial strain is *Propionobacterium acnes;* and/or the bacterial strain is selected from among the *Corynebacterium spp* family, preferably the *Corynebacterium xerosis* bacterial strain; *and*/*or* the bacterial strain is selected from among the *Staphylococcus spp* family, preferably the bacterial strain is *Staphylococcus epidermis;* and **in that** the fungal strain is selected from among the *Malassezia spp* family, preferably the fungal strain is *Malassezia furfur.*
